(19) **Europäisches Patentamt / European Patent Office / Office européen des brevets**

(11) **EP 4 591 817 A1**

(12) **EUROPEAN PATENT APPLICATION**
published in accordance with Art. 153(4) EPC

(43) Date of publication:
**30.07.2025 Bulletin 2025/31**

(21) Application number: **23868591.1**

(22) Date of filing: **20.09.2023**

(51) International Patent Classification (IPC):
**A61B 34/10** (2016.01)    **A61B 34/30** (2016.01)
**A61B 34/00** (2016.01)

(52) Cooperative Patent Classification (CPC):
**A61B 34/00; A61B 34/10; A61B 34/30**

(86) International application number:
**PCT/KR2023/014301**

(87) International publication number:
**WO 2024/063539 (28.03.2024 Gazette 2024/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.09.2022 KR 20220121028**

(71) Applicant: **Curexo Inc.
Seoul 05814 (KR)**

(72) Inventors:
• **LEE, Jae Jun
Seoul 05814 (KR)**
• **KIM, Bong Oh
Seoul 05814 (KR)**

(74) Representative: **Stöckeler, Ferdinand et al
Schoppe, Zimmermann, Stöckeler
Zinkler, Schenk & Partner mbB
Patentanwälte
Radlkoferstrasse 2
81373 München (DE)**

(54) **APPARATUS FOR PLANNING CUTTING PATH OF SURGICAL ROBOT, AND METHOD THEREOF**

(57)    Disclosed are an apparatus for planning a cutting path of a surgical robot and a method thereof, the method of planning the cutting path of the surgical robot, including: determining a position and posture of an implant to be installed in a surgical target bone based on a 3D model of a surgical target bone; determining a cutting surface of the surgical target bone required to be cut for installation of the implant; determining a cutting start position where the surgical robot starts cutting for the cutting surface, and an entrance direction of the surgical robot in the cutting start position; setting a maximum region in which the cutting path by the surgical robot is definable based on the cutting start position and the entrance direction; generating a modified 3D model in which a virtual cut state of the cutting surface is reflected; and creating the cutting path of the surgical robot starting from the cutting start position within a range of the maximum region based on the modified 3D model.

Thus, a patient-tailored cutting path is automatically created in consideration of the bone diversity of patients, thereby improving a surgical effect.

[Fig.3]

Start

Plan position and posture of implant — S10

Determine cutting surface of surgical target bone — S20

Set safety region and free cutting region for each cutting surface — S30

Determine cutting start position, entrance direction and posture of surgical robot — S40

Set maximum region where cutting path can be defined — S50

Generate modified 3D model in which virtual cut state is reflected — S60

Create cutting path of surgical robot — S70

End

**Description**

[TECHNICAL FIELD]

The disclosure relates to an apparatus for planning a cutting path of a surgical robot and a method thereof, and more specifically to an apparatus for planning a cutting path for a surgical target bone to be cut by a surgical robot to install an implant during artificial joint replacement surgery using the surgical robot.

[BACKGROUND ART]

Robotic surgery is broadly divided into passive robotic surgery, semi-active robotic surgery, and active robotic surgery. While the passive robotic surgery refers to a method in which a surgeon controls a robot in person throughout the entire surgical process, the semi-active robotic surgery involves a surgeon in performing cutting and the like processes using the robot, during which the movement of a surgical tool by the surgeon is restricted by a robot to a predetermined path based on haptic feedback. Meanwhile, active robotic surgery refers to a method in which the robot automatically performs surgery according to a planned cutting path without the intervention of a surgeon.

As above, in the active robotic surgery, the robot actively cuts a bone, and it is therefore important to plan the cutting path suitable for an implant to be installed.

In the related art, a library in which the cutting paths are defined according to the shapes or sizes of the implant is prepared in advance. When a planner determines the shape and size of the implant to be installed in a surgical target bone, the cutting path corresponding to the determined shape and size of the implant is selected and utilized from the library. However, such a library-based cutting path does not take into account the diversity of bone shapes for each patient, and thus has limitations in providing patient-tailored surgery.

Further, the cutting path is usually created for an area slightly smaller than the implant leaving a predetermined cutting margin to prevent damage to soft tissue, and therefore a considerable portion of a residual bone region remains uncut. This residual bone region not only causes inconvenience because a doctor should do additional cutting and final trimming in person, but also causes a delay in surgical time. The cutting margin for minimizing the residual bone region varies depending on the specific positions of the implant, but it is difficult to set the conventional library-based cutting path in consideration of all the cases of the minimum cutting margin according to various positions of the implant, thereby having a problem that a large number of residual bone regions remain.

[DISCLOSURE]

[TECHNICAL PROBLEM]

The disclosure has been conceived to solve the problems described as above, and an aspect of the disclosure is to provide an apparatus for planning a cutting path of a surgical robot, which can create a patient-tailored cutting path in consideration of bone diversity of patients and minimize a residual bone region, and a method thereof.

[TECHNICAL SOLUTION]

The aspect of the disclosure may be achieved by a method of planning a cutting path of a surgical robot, the method including: determining a position and posture of an implant to be installed in a surgical target bone based on a 3D model of a surgical target bone; determining a cutting surface of the surgical target bone required to be cut for installation of the implant; determining a cutting start position where the surgical robot starts cutting for the cutting surface, and an entrance direction of the surgical robot in the cutting start position; setting a maximum region in which the cutting path by the surgical robot is definable based on the cutting start position and the entrance direction; generating a modified 3D model in which a virtual cut state of the cutting surface is reflected; and creating the cutting path of the surgical robot starting from the cutting start position within a range of the maximum region based on the modified 3D model.

In this case, the method may further include setting a safety region to protect tissue around the surgical target bone, wherein the cutting path is created not to perform cutting in the safety region.

Meanwhile, one or more cutting start positions of the surgical robot may be determined for the cutting surface based on skin incision information preset for the cutting of the cutting surface and a location of the safety region, and the maximum region and the cutting path may be individually determined corresponding to each cutting start position.

Further, the cutting path may be individually created for each of the cutting surfaces based on the number of cutting surfaces required to be cut for the installation of the implant.

In addition, the method may further include setting a free cutting region that the surgical robot can perform cutting freely based on a location of tissue around the surgical target bone and preset skin incision information.

Meanwhile, the creating the cutting path of the surgical robot may include: identifying an outer boundary of the cutting surface in the modified 3D model; setting a cutting margin region excluded from the cutting by applying a predetermined offset from the outer boundary; determining a cutting path creation region to create the cutting path based on the maximum region and the cutting margin region; and creating the cutting path with the cutting path creation region served as a boundary.

In addition, the method may further include determining whether the surgical robot can perform cutting for all regions within the cutting path creation region in a predetermined range of postures, wherein the creating the cutting path includes, upon the cutting not performed for all the regions within the cutting path creation region in the predetermined range of postures, creating the cutting path so that the surgical robot can cut some regions within the cutting path creation region, then return to the cutting start position, and complete the cutting by changing the posture and entering the position again.

Further, one or more cutting start positions of the surgical robot may be determined for the cutting surface based on skin incision information preset for the cutting of the cutting surface and a location of the safety region, and a plurality of cutting path creation regions may be determined corresponding to the plurality of cutting start positions, respectively.

In addition, the creating the cutting path of the surgical robot may include determining a return position for the surgical robot to return to the cutting start position after the cutting.

Further, the cutting path may be determined considering types of cutting tool of the surgical robot and cutting characteristics of the cutting tool.

In addition, the determining the position and posture of the implant may include determining the position and posture of the implant based on a user input made through a user interface unit.

The aspect of the disclosure may be achieved by a surgical robot cutting path planning apparatus for planning a cutting path of a surgical robot, the apparatus including a processor, the processor being configured to: determine a position and posture of an implant to be installed in a surgical target bone based on a 3D model of a surgical target bone; determine a cutting surface of the surgical target bone required to be cut for installation of the implant; determine a cutting start position where the surgical robot starts cutting for the cutting surface, and an entrance direction of the surgical robot in the cutting start position; set a maximum region in which the cutting path by the surgical robot is definable based on the cutting start position and the entrance direction; generate a modified 3D model in which a virtual cut state of the cutting surface is reflected; and create the cutting path of the surgical robot starting from the cutting start position within a range of the maximum region based on the modified 3D model.

[ADVANTAGEOUS EFFECTS]

As described above, according to the disclosure, a patient-tailored cutting path is automatically created in consideration of the bone diversity of patients, thereby improving a surgical effect.

Further, according to the disclosure, a cutting path is created to minimize a residual bone region remaining after cutting by a surgical robot, thereby simplifying a surgical process and effectively preventing surgical delays.

[DESCRIPTION OF DRAWINGS]

FIG. 1 is a diagram showing schematic configurations of a joint replacement robotic surgery system including a cutting path planning apparatus according to an embodiment of the disclosure;

FIG. 2 is a block diagram showing detailed configurations of a cutting path planning apparatus according to an embodiment of the disclosure;

FIG. 3 is a flowchart showing a surgical robot cutting path planning method of a cutting path planning apparatus according to an embodiment of the disclosure;

FIGS. 4 and 5 are reference views for illustrating that a cutting path planning apparatus according to an embodiment of the disclosure sets a safety region and a free cutting region;

FIGS. 6 and 7 are reference views for illustrating that a cutting path planning apparatus according to an embodiment of the disclosure sets a maximum region;

FIG. 8 is a flowchart showing how a cutting path planning apparatus according to an embodiment of the disclosure creates a cutting path of a surgical robot;

FIG. 9 is a reference view for illustrating that a cutting path planning apparatus according to an embodiment of the disclosure sets an outer boundary of a cutting surface and a cutting margin region;

FIGS. 10 and 11 are reference views for illustrating that a cutting path planning apparatus according to an embodiment of the disclosure sets a cutting path creation region;

FIGS. 12 and 13 are reference views for illustrating that a cutting path planning apparatus according to an embodiment of the disclosure creates a cutting path based on a cutting path creation region; and

FIG. 14 is a reference view for illustrating a coordinate transformation process for a cutting path planned through a cutting path planning apparatus according to an embodiment of the disclosure.

[BEST MODE]

Below, specific embodiments of the disclosure will be described with reference to the drawings. However, detailed descriptions of known functions or configurations that may obscure the gist of the disclosure in the following description and the accompanying drawings will be omitted. Further, it should be noted that like numerals refer to like elements identical components are indicated with the same drawing reference numerals throughout the drawings.

A surgical robot cutting path planning apparatus according to the disclosure plans a cutting path for a surgical target bone to be cut by a surgical robot to install an implant during artificial joint replacement surgery. The artificial joint replacement surgery described in this specification includes total knee replacement surgery, partial knee replacement surgery, and hip replacement surgery. Hereinafter, the total knee replacement surgery of the artificial joint replacement surgery will be described as an example.

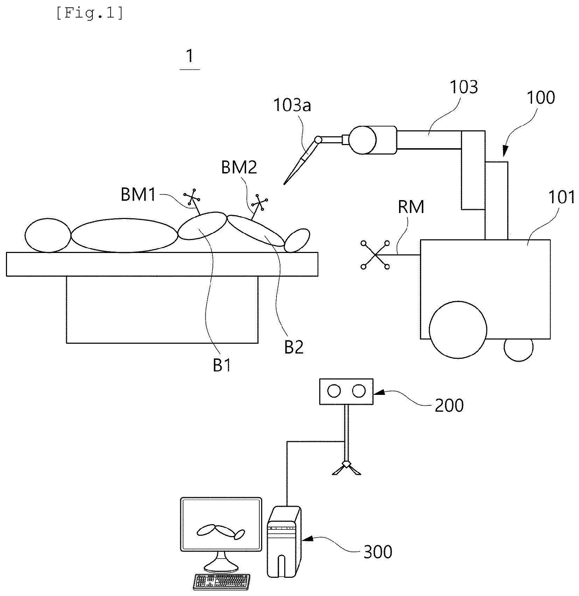

FIG. 1 is a diagram showing schematic configurations of a joint replacement robotic surgery system 1 including a surgical robot cutting path planning apparatus according to an embodiment of the disclosure;

Referring to FIG. 1, a joint placement robotic surgery system 1 placed in a surgical site includes a bone markers BM1 and BM2 fixed to surgical target bones B1 and B2, a surgical robot 100, a tracking apparatus 200, and a surgical robot cutting path planning apparatus 300 (hereinafter referred to as a 'cutting path planning apparatus').

The surgical target bones B1 and B2 refer to bones that are the targets of robotic surgery, and FIG. 1 shows a femur B1 and a tibia B2 as examples. The bone markers BM1 and BM2 that serve as references for tracking the positions of the femur B1 and the tibia B2 during surgery are fixedly installed on the femur B1 and the tibia B2, respectively.

The surgical robot 100 refers to a robot that performs surgery for the joint replacement, and includes a robot base 101 and a robot arm 103. Various surgical tools 103a, including a bur, for cutting bones, etc., may be attached to an end effector, i.e., the end of the robot arm 103. Further, the surgical robot 100 may be mounted with various sensors capable of sensing state information of the surgical robot 100, such as a load applied to the attached surgical tool, and an operating speed of the robot. A robot marker RM, which serves as a reference for tracking the position of the surgical robot 100 during surgery, is fixed to the base 101 of the surgical robot 100.

For reference, passive or active type optical markers may be used as the bone markers BM1 and BM2 and the robot marker RM. The optical marker includes a plurality of bar members shaped like branches branching off in different directions with respect to a center point, and a ball marker may be formed at the end of each bar. This is merely an example of the shape of the optical marker, and the optical marker may be implemented in various other known shapes.

The tracking apparatus 200 is to track the positions and postures of the bone markers BM1 and BM2 fixed to the surgical target bones B1 and B2 and the robot marker RM fixed to the surgical robot 100, and may be implemented as an optical tracking system (OTS). For reference, the optical tracking system refers to a system that may track the position and posture in a three-dimensional space in real time by tracking the marker by two infrared cameras and converting the distance by triangulation. The tracking principle of such optical tracking system is widely known, and thus detailed descriptions thereof will be omitted for simplicity.

The cutting path planning apparatus 300 plans the cutting path of the surgical robot 100 for the surgical target bones B1 and B2 required to be cut to install an implant in the surgical target bones B1 and B2 during joint replacement surgery. The planning for the cutting path may be performed before the robot surgery or may be performed according to change in the plan during the surgery. The cutting path includes information about a position to which the surgical robot 100 is required to move when performing the cutting and a posture of the surgical robot 100 at the corresponding position. The cutting path may be created as a relative position of the surgical robot 100 based on an implant coordinate system and the posture of the surgical robot 100 during the cutting process.

The cutting path planning apparatus 300 may be implemented including a computer (processor) that plans the cutting path, a display that displays the planned cutting path, and a memory that stores information about the created cutting path. For reference, FIG. 1 illustrates that the cutting path planning apparatus 300 is implemented as a separate apparatus physically separated from the surgical robot 100, but in some cases, the processor and the memory of the cutting path planning apparatus 300 may be provided in the surgical robot 100, and the display and the tracking apparatus 200 may be installed and connected together so that they can exchange various pieces of information through a communication module.

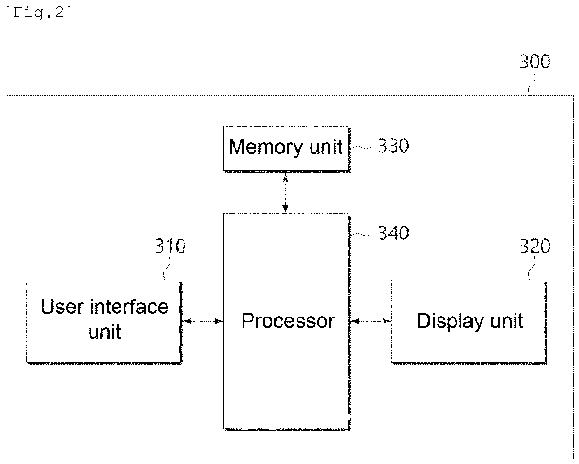

FIG. 2 is a block diagram showing detailed configurations of the cutting path planning apparatus 300 according to an embodiment of the disclosure. Referring to FIG. 2, the cutting path planning apparatus 300 according to an embodiment of the disclosure includes a user interface unit 310, a display unit 320, a memory unit 330, and a processor 340.

The user interface unit 310 refers to a module for receiving various inputs from a user in the process of planning the cutting path of the surgical robot 100, and may be implemented by various input devices such as a mouse, a keyboard, a keypad, a button, and a pendant.

The display unit 320 is to display various pieces of information including images, graphics, texts, etc. on a screen thereof, and may be implemented as a liquid crystal display (LCD) panel, a light emitting diode (LED) panel, an organic light emitting diode (OLED) panel, etc. Further, the user input unit 310 and the display unit 320 may be integrated and implemented as a single device such as a touch screen. The display unit 320 displays various graphic user interfaces (GUI) and planning outputs provided in the process of planning the position and posture of an implant and the process of planning the cutting path of the surgical robot 100, including an implant model, a 2D image and 3D model of a surgical target bone, the cutting path of the surgical robot 100, etc.

The memory unit 330 is implemented as a memory device such as a random-access memory (RAM), a flash memory, an erasable programable read-only memory (EPROM), etc., and may be configured to store various operating systems (OS), middleware, platforms, and various applications of the cutting path planning apparatus 300 and to store program codes, processed image signals, audio signals, and various pieces of data. Further, the memory unit 330 is configured to store a patient's computed tomography (CT) and magnetic resonance imaging (MRI) images taken before surgery, an implant library of implant attribute information such as the implant type/shape/length/size, etc., information about the cutting tool mounted to the robot arm of the surgical robot 100 including types of a cutting tool and the cutting characteristics such as a cutting depth and a cutting width, and various pieces of reference information applied in the process of planning the cutting path of the surgical robot 100.

The processor 340 executes a program code stored in the apparatus to perform the cutting path planning procedure for the surgical robot 100 based on a user input made from the user interface unit 310 and various stored reference information.

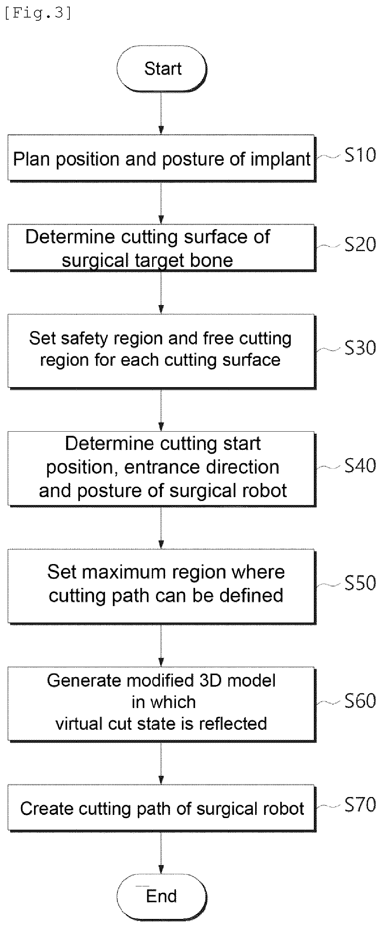

Below, a surgical robot cutting path planning method performed by the cutting path planning apparatus 300 will be described with reference to FIG. 3.

FIG. 3 is a flowchart showing a surgical robot cutting path planning method of the cutting path planning apparatus 300 according to an embodiment of the disclosure.

Referring to FIG. 3, the processor 340 determines the position and posture of an implant to be installed in a surgical target bone based on a 3D model of the surgical target bone (S10). Here, the 3D model of the surgical target bone refers to a model in which the surgical target bone is reconstructed in three dimensions based on a medical image of the surgical target bone taken before surgery, such as a CT image or an MRI image. The 3D model of the surgical target bone becomes the basis for planning the cutting path of the surgical robot.

The position and posture of the implant may be determined based on a user's input made through the user interface unit 310. When a user selects an implant to be installed in the surgical target bone from the implant library, the processor 340 creates a virtual model of the selected implant and displays the virtual model superimposed on a 3D bone model, so that an appropriate installation position and posture, such as an installation angle, of the implant can be determined based on a user input for moving the virtual model of the implant on the 3D bone model through the user interface unit 310.

Meanwhile, as described above, the position and posture of the implant may be automatically determined based on preset implant installation criteria without relying entirely on the user input. For example, the processor 340 may be implemented to identify the position of a predetermined landmark in the 3D bone model and the shape, size, etc. of the corresponding landmark, and determine the type of an implant and the appropriate position and posture of the implant according to the implant installation criteria information corresponding to the identified results.

When the implant planning procedure for determining the installation position and posture of the implant is completed, a cutting surface of the surgical target bone is determined according to the foregoing planning results (S20). The cutting surface refers to a surface of the surgical target bone required to be cut for the installation of the implant, and the criteria for determining the cutting surface may be preset and stored corresponding to the characteristics of the implant, such as the type, shape and size of the implant. In this case, a single or multiple cutting surfaces may be determined, and the cutting surface may be cut not only flat but also in curved shape as a patient-tailored form. The determination of the cutting surface may include determining the position of the cutting surface, the number of cutting surfaces, the shape of the cutting surface, and the cutting order of the cutting surfaces.

Next, the processor 340 sets a safety region and a free cutting region for each cutting surface (S30). For reference, the safety region and the free cutting region refer to regions that serve as references when creating the cutting path, and may be set based on an implant coordinate system (to be described later). Further, the safety region and the free cutting region are related to a location where the implant is to be installed, and thus the location information of the safety region and the free cutting region may be set in advance and included as additional information to the implant shape information. Accordingly, when the implant to be installed in the surgical target bone is selected in the implant planning procedure, the safety region and the free cutting region may be automatically set corresponding to the selected implant.

The safe region refers to a region around which soft tissue such as ligaments, blood vessels, muscles, and skin are located, and should not be invaded when cutting is performed by the surgical robot 100. In order to protect the tissue around the surgical target bone, the safe region is excluded from the cutting path creation region when the cutting path is created, so that the cutting cannot be performed in the safe region.

When the safety region is set based on the implant coordinate system, the safety region may be set based on a user input made through the user interface unit 310, or may be automatically set by the processor 340 based on inputs of information about the location of soft tissue such as ligaments, blood vessels and muscles, and skin incision information such as the location of the skin to be incised during robot surgery and the length of incision, or based on prestored information.

Meanwhile, the free cutting region refers to a region where allows the surgical robot 100 to perform cutting freely without concerning about damage to the surrounding tissue. There is no soft tissue around the free cutting region by incising the skin so there is no concern about damage to the surrounding tissue when cutting the bone in the free cutting region during robotic surgery. As will be described below, the cutting path is created leaving a predetermined cutting margin region in order to prevent damage to soft tissue such as skin, but there is no need to leave the cutting margin region in the free cutting region.

Like the safety region, the free cutting region may be set by a user input made through the user interface unit 310 or may be automatically set by the processor 340 based on the information about the location of soft tissue and the skin incision information.

Meanwhile, when the planning of the cutting path is performed during robotic surgery, a user may designate the free cutting region in person using a probe of which the position is trackable by the tracking apparatus 200. Further, an affected area image captured by a camera included in the tracking apparatus 200 or a separate camera may be processed to recognize a skin incision region, and the free cutting region may be set based on the recognized skin incision region.

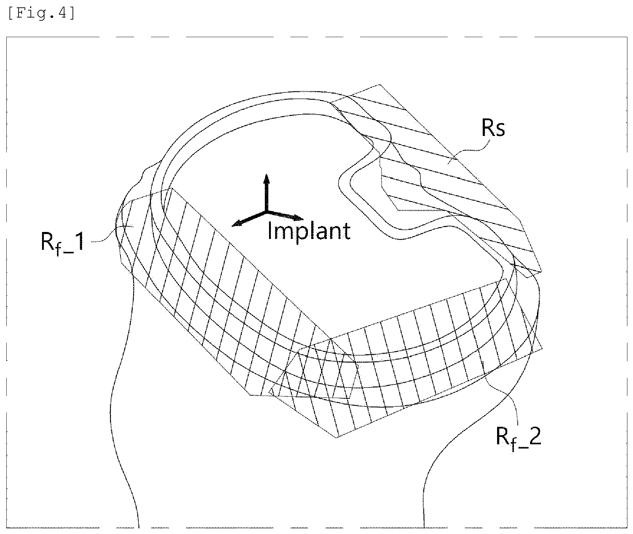

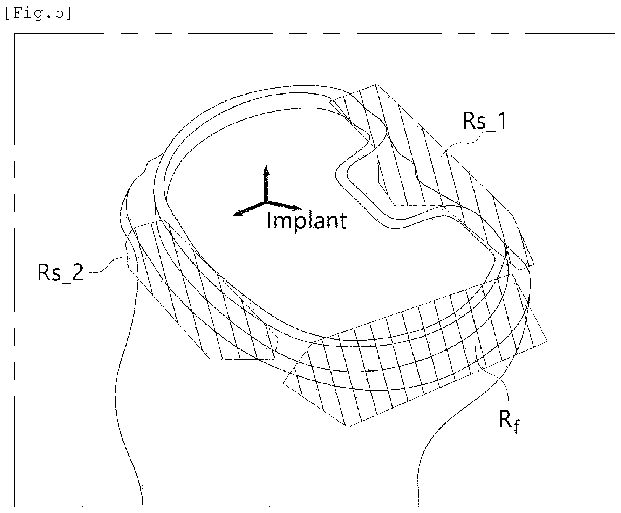

FIGS. 4 and 5 are reference views for illustrating that the cutting path planning apparatus 300 according to an embodiment of the disclosure sets the safety region and the free cutting region, which are a first example and a second example showing that a safety region $R_s$ and a free cutting region $R_f$ are set for one cutting surface.

Referring to FIGS. 4 and 5, the safe region $R_S$ and the free cutting region $R_f$ may be set for each cutting surface, and a single or multiple regions may be set on one cutting surface. Meanwhile, regions having the same attributes may be set to overlap each other. For example, FIG. 4 shows that two adjacent free cutting regions $R_f\_1$ and $R_f\_2$ are set to overlap partially each other.

Next, the processor 340 determines a cutting start position at which the surgical robot 100 starts cutting the surgical target bone for each cutting surface, and an entrance direction and posture of the surgical robot at that cutting start position (S40).

Based on the skin incision information during the cutting, such as skin incision location and incision length, which is preset or input by a user, and based on the locations of the safety region and the free cutting region, one or more cutting start positions of the surgical robot 100 may be determined with respect to one or more cutting surfaces.

The criteria for determining the cutting start position and the entrance direction/posture of the surgical robot 100 may be determined by a user input made through the user interface unit 310 or may be based on criteria preset corresponding to the cutting surface. In other words, the positions and directions/postures in which the surgical robot 100 can safely enter, and the optimal number thereof may be preset corresponding to the type of the cutting surface, the position, area, shape, and angle of the cutting surface, the skin incision information, etc. For example, a plurality of cutting start positions may be set if it is difficult to enter once and cut all regions when the area of the cutting surface, the shape of the cutting surface, the skin incision information, and a motion range of the surgical robot 100 such as the posture change angle range of the surgical robot 100, are comprehensively taken into account.

Meanwhile, when the planning of the cutting path is performed during robotic surgery, a position designated by a user may be applied as the cutting start position by using a probe of which the position is trackable by the tracking apparatus 200. Further, an affected area image captured by a camera included in the tracking apparatus 200 or a separate camera may be processed to recognize a position where there is no concern about damage to soft tissue or collision with a bone or the like upon the entry of the surgical robot 100, and the recognized position may be set as the cutting start position.

Next, the processor 340 sets a maximum region where the cutting path of the surgical robot 100 can be defined (S50). The maximum region is also individually generated for each cutting surface. The maximum region refers to a maximum limit range where the cutting path can be created, and is applied as a criterion for limiting a creation range of the cutting path so that the cutting path beyond the maximum region cannot be created when being created in the future.

This maximum region may be individually set for each cutting start position based on the cutting start position set in the previous step and the entrance direction/posture of the surgical robot, and the maximum region may be determined considering the characteristics of the surgical target bone, such as the size and shape of the surgical target bone, and the characteristics of the implant, such as the size and shape of the implant.

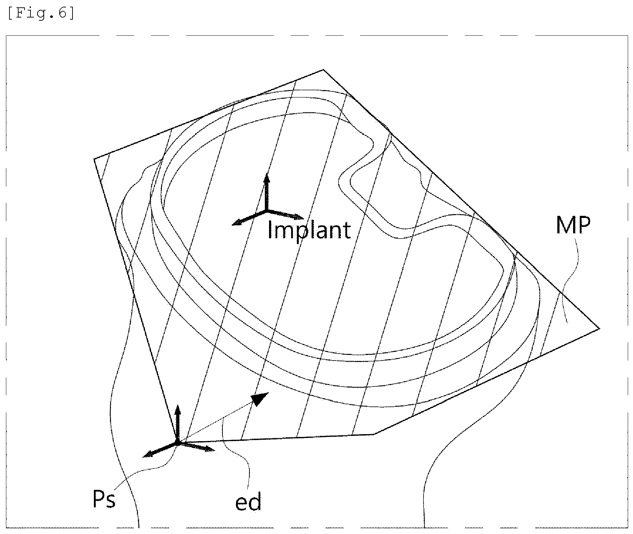

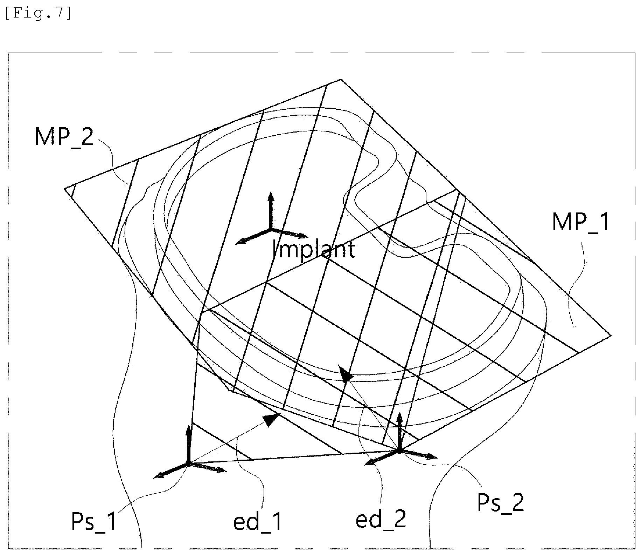

FIGS. 6 and 7 are reference views for illustrating that the cutting path planning apparatus 300 according to an embodiment of the disclosure sets the maximum region, which are a first example and a second example showing that the maximum regions are set, respectively.

Referring to FIGS. 6 and 7, a maximum region MP may be defined in the form of a plane extending from the cutting start position Ps in consideration of the entrance direction/posture of the surgical robot 100, and the maximum region MP may be determined in various shapes based on a distance between the surgical target bone and the cutting start position Ps, the entrance direction/posture of the surgical robot 100, the characteristics of the surgical target bone and the implant, etc.

The maximum region MP is formed to be larger than the size of the implant and the surgical target bone. Further, a plurality of maximum regions MP may be set corresponding to the cutting start positions Ps, and the plurality of maximum regions MP may have overlapping regions.

Referring to FIG. 7, when two cutting start positions Ps_1 and Ps_2 and the entrance directions ed_1 and ed_2/postures corresponding to the cutting start positions Ps_1 and Ps_2 are determined for one cutting surface, a first maximum region MP_1 corresponding to the first cutting start position Ps_1 and a second maximum region MP_2 corresponding to a second cutting start position Ps_2 are generated, respectively. In this case, as shown in FIG. 7, the first maximum region MP_1 and the second maximum region MP_2 may have some overlapping regions.

For reference, the flowchart of FIG. 3 discloses that the maximum region is generated after generating the safe region and the free cutting region, but is not limited to this order. Of course, the safe region and the free cutting region may be generated after generating the maximum region.

Next, the processor 340 generates a modified 3D model in which the state in which the cutting surface has been virtually cut is reflected (S60). The modified 3D model refers to a 3D model generated by predicting the state after cutting of each cutting surface based on the 3D model of the surgical target bone before cutting, although the surgical target bone has not actually been cut by the surgical robot 100. The modified 3D model may be generated by predicting the virtual cut state of each cutting surface based on the characteristics of the implant to be installed in the surgical target bone, such as the position and posture of the implant, and the shape and size of the implant, and the cutting characteristics of a cutting tool for performing the cutting, such as the depth of one cutting and a cutting width.

The modified 3D models are respectively generated corresponding to the number of cutting surfaces in consideration of the planned cutting order for one or more cutting surfaces. Taking the cutting order into account, when there is another cutting surface that has already cut before cutting the present cutting surface, a modified 3D model may be generated with the cut state of the present cutting surface including the already cut state of the other cutting surface. Accordingly, for the first cutting surface to be cut first, the first modified 3D model, in which the virtual cut state of the first cutting surface is reflected based on an initial 3D model of the surgical target bone, is generated. In the case of the modified 3D models that do not corresponding to the first cutting surface, the modified 3D models, in which the virtual cut state of the present cutting surface is additionally reflected based on the modified 3D model that has accumulatively reflected the cutting surfaces previously cut in the cutting order, i.e., based on the modified 3D model that has been immediately previously generated, are generated in sequence.

Next, the processor 340 creates the cutting path of the surgical robot 100 starting from the cutting start position within the range of the maximum region based on the modified 3D model (S70). The cutting path may be individually created for each cutting surface corresponding to the number of cutting surfaces to be cut for the installation of the implant, and may be variously defined as a straight line, a curved line, or a combination thereof. The cutting path may include not only a movement path of the surgical robot 100 but also posture information of the surgical robot 100 on that path.

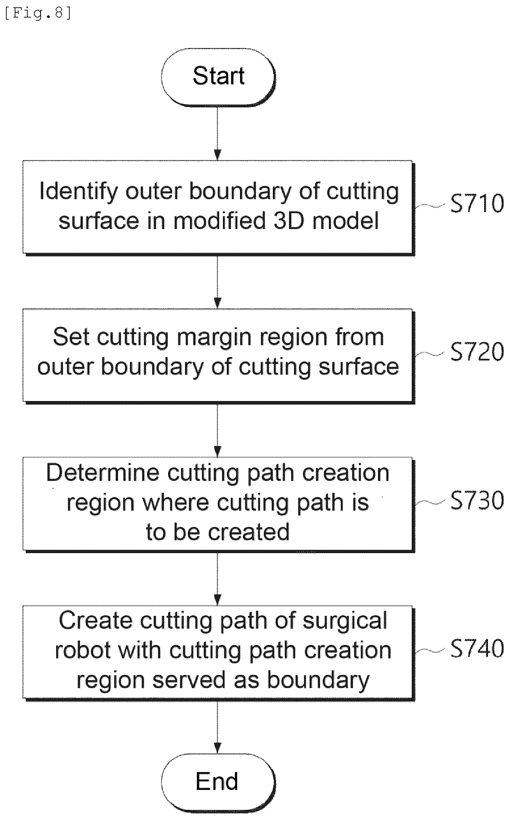

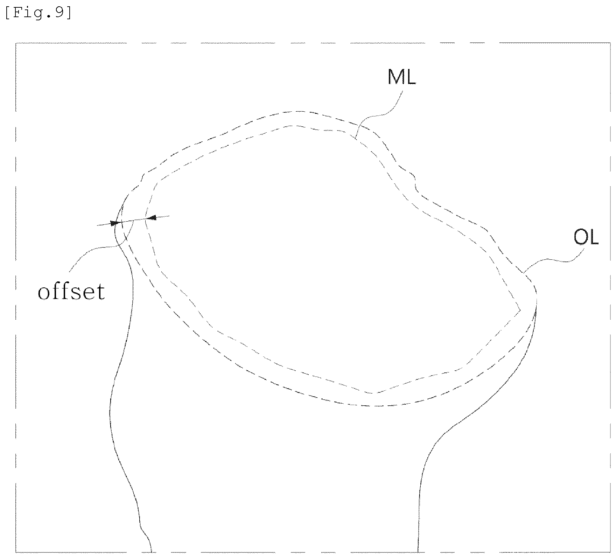

FIG. 8 is a flowchart showing how the cutting path planning apparatus 300 according to an embodiment of the disclosure creates the cutting path of the surgical robot 100, and FIGS. 9 to 13 are reference views for illustrating that the cutting path planning apparatus 300 according to an embodiment of the disclosure creates the cutting path of the surgical robot 100. Below, the detailed operations of step S70 in FIG. 3 will be described with reference to FIGS. 8 to 13.

First, the processor 340 identifies an outer boundary OL of the cutting surface in the modified 3D model (S710). The processor 340 may identify the outer boundary OL of the cutting surface by recognizing the outermost edge of the surgical target bone on the cutting surface in the modified 3D model corresponding to the cutting surface in order to create the cutting path for each cutting surface.

Next, the processor 340 sets a cutting margin region ML by applying a predetermined offset from the outer boundary OL of the cutting surface (S720). Referring to FIG. 9, the cutting margin region ML refers to a region excluded from cutting to protect the soft tissue, and may be set as a region that is located a predetermined distance inside the surgical target bone from the outer boundary OL. A preset value may be applied to the size of the offset for the location inside the bone, and may be set to vary depending on the types of cutting tool.

Next, the processor 340 determines the cutting path creation region CR for creating the cutting path based on the outer boundary OL and the cutting margin region ML of the cutting surface and the safety region $R_S$ and the free cutting region $R_f$ set in previous step (S730). The cutting path creation region CR refers to a range in which the cutting path is to be actually generated, and the cutting path is generated so that all cutting is performed for the cutting path creation region CR.

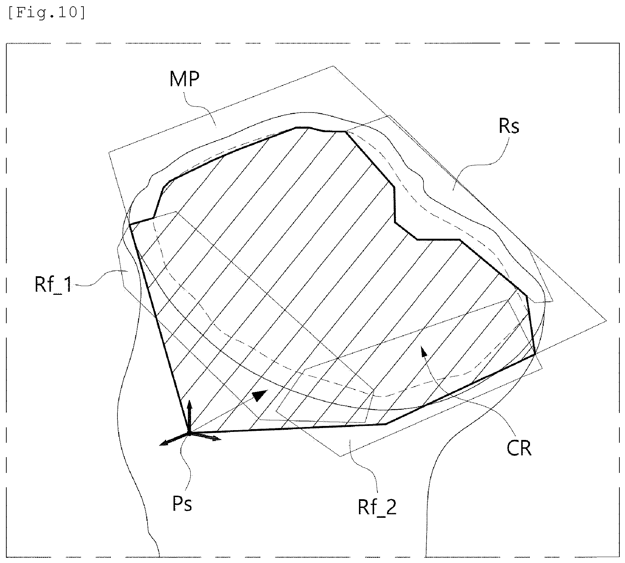

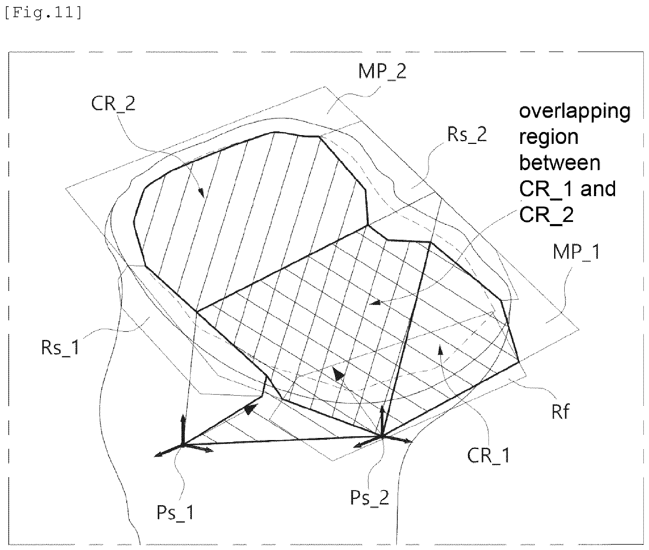

FIGS. 10 and 11 are reference views for illustrating that the cutting path planning apparatus 300 according to an embodiment of the disclosure sets a cutting path creation region, which are a first example and a second example of the cutting path creation region CR set by the cutting path planning apparatus 300, respectively.

Referring to FIG. 10 and FIG. 11, the cutting path creation region CR is determined to satisfy the following conditions. The cutting path creation region CR includes the cutting start position Ps as a vertex, is defined within the maximum region MP, and is determined not to invade the safety region $R_S$. Meanwhile, the cutting path creation region CR is basically created without passing the boundary of the cutting margin region ML in a place where the cutting margin region ML does not overlap with the safety region $R_S$, but the cutting path creation region CR may be created including the outer boundary of the bone without being limited by the cutting margin region ML in the free cutting region $R_f$.

Meanwhile, as shown in FIG. 11, the cutting path creation regions CR_1 and CR_2 may be individually generated corresponding to the cutting start positions Ps_1 and Ps_2, respectively. In this case, considering that a plurality of cutting path creation regions CR_1 and CR_2 overlap each other, the cutting path creation regions CR_1 and CR_2 may be generated to minimize the overlapping regions or may be modified by a user. This is to prevent the cutting paths from being repeatedly created for the overlapping regions of the cutting path creation regions.

In this way, when the cutting path creation region CR is generated, the processor 340 creates the cutting path of the surgical robot 100 by using the cutting path creation region CR as the boundary (S740). The cutting path may be generated to start from the cutting start position, and may be determined including a return position for the surgical robot 100 to return to the cutting start position after cutting.

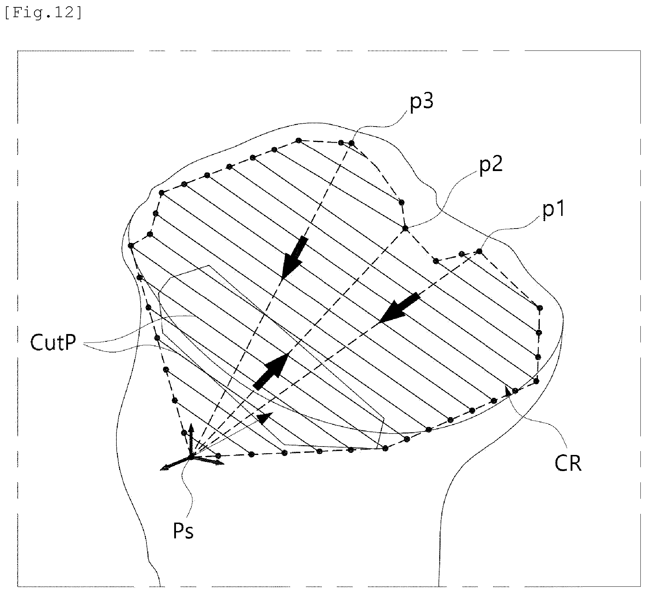

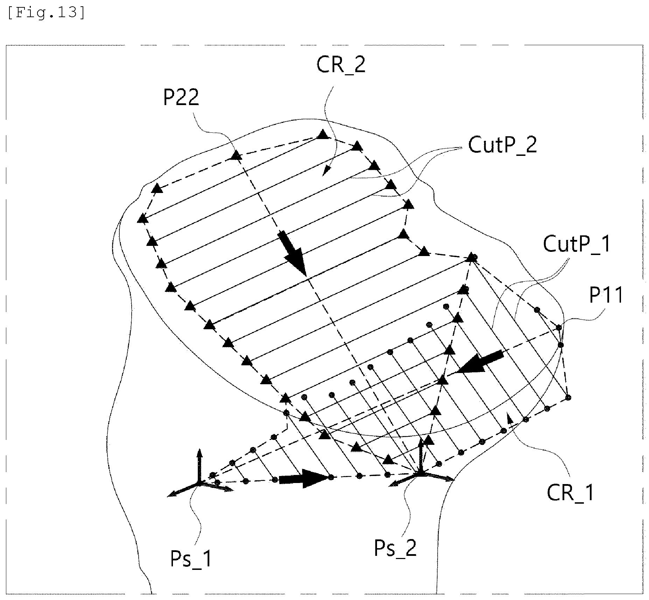

FIGS. 12 and 13 are reference views for illustrating that the cutting path planning apparatus 300 according to an embodiment of the disclosure creates a cutting path based on a cutting path creation region, which are a first example and a second example of a cutting path CutP created by the cutting path planning apparatus 300, respectively.

Referring to FIGS. 12 and 13, the cutting path CutP is created so that cutting is performed for all regions within the cutting path creation region CR. The cutting path CutP starts from the cutting start position Ps and is created reflecting the entrance direction/posture of the surgical robot 100. Further, a path interval, etc. may be varied depending on the types of cutting tool to be used for the cutting and the characteristics of the cutting tool, such as the depth of one cutting, a cutting width, etc.

Meanwhile, the processor 340 may determine whether the surgical robot 100 can perform cutting for all regions within the cutting path creation region CR in a predetermined range of postures by considering the characteristics of the surgical robot 100, such as the posture change range and motion range of the robot arm of the surgical robot 100, and the characteristics of the cutting tool. If the surgical robot 100 cannot perform the cutting for all regions within the cutting path creation region CR with one posture or posture change in a predetermined range, the cutting path CutP may be created so that the surgical robot 100 can cut some regions within the cutting path creation region CR, then return to the cutting start position Ps and complete the cutting by changing the posture and entering the position again. FIG. 12 shows an example that the cutting path CutP is created so that the surgical robot 100 can perform cutting, return from p1 to the cutting start position Ps, re-enter p2 from the cutting start position Ps, perform subsequent cutting on regions that have not been cut yet, and finally return from p3 to the cutting start position Ps after completing the cutting.

Meanwhile, FIG. 13 shows an example in which the cutting paths CutP_1 and CutP_2 are individually created corresponding to the plurality of cutting path creation regions CR_1 and CR_2. In this way, when the cutting region is divided for one cutting surface to create the cutting path, the cutting order for the divided cutting regions may also be determined together. FIG. 13 shows an example that the first cutting path CutP_1 is created starting from the first cutting start position Ps_1, completing the cutting for a portion of the first the cutting path creation region CR_1, and initially returning to p11, and then the second cutting path CutP_2 is created moving the surgical robot 100 to the second cutting start position Ps_2, completing the cutting for the second cutting path creation region CR_2, and finally returning to p22.

In this case, as shown in FIG. 13, the cutting path may be created so that the surgical robot 100 does not repeatedly pass through part or all of the overlapping regions between the first cutting path creation region CR_1 and the second cutting path creation region CR_2. To prevent the surgical robot 100 from unnecessary movement once the overlapping region has already been cut through the cutting path corresponding to one of the cutting path creation regions, the cutting path is created so that cutting can be started from the part where the cutting has not been performed yet.

Although FIGS. 12 and 13 show examples that the cutting path is created based on a combination of straight paths, the shape of the cutting path may be variously defined in the form of a straight line, a curved line, or a combination thereof as described above. For example, when the skin incision region is so narrow that it is difficult for the rectilinear motion in the left and right directions to cut the entire region required to be cut as shown in FIGS. 12 and 13, the cutting path may be created in the form of an arc for left and right motion rotating about a specific position.

For reference, FIGS. 4 to 13 show that the cutting path is planned for one cutting surface, but the cutting path may be planned even for other cutting surfaces in the same manner.

The foregoing planning of the cutting path of the surgical robot may be performed in a pre-surgical planning step before the surgery, or in a plan changing step during the surgery. For reference, when the planning of the cutting path is performed in the plan changing step during the surgery, the cutting path may be planned for all the cutting surfaces at once and then the cutting may be performed, or the cutting path may be created for each cutting surface and the cutting for the corresponding cutting surface may be performed simultaneously.

As described above, the cutting path of the surgical robot 100 is planned based on the implant coordinate system and the surgical robot 100 operates based on the surgical robot coordinate system, and therefore these coordinate systems are required to undergo a matching process.

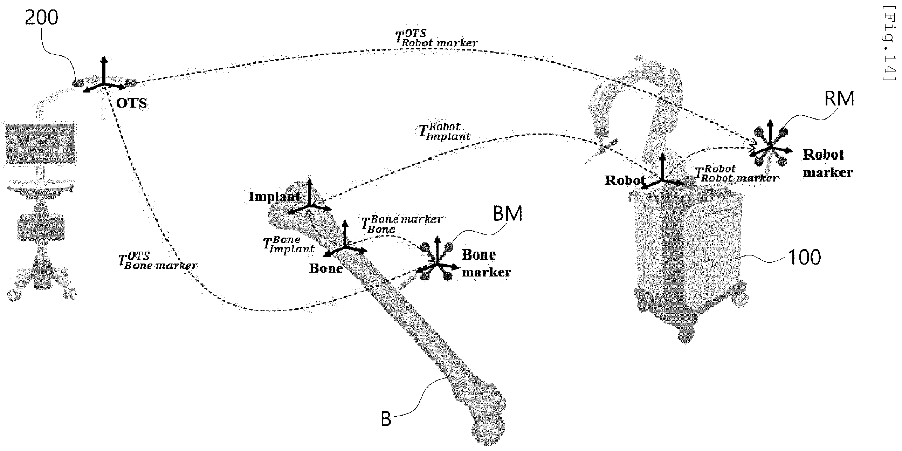

FIG. 14 is a reference view for illustrating a coordinate transformation process for a cutting path to use the planned cutting path to the robotic surgery according to an embodiment of the disclosure.

As described above, in the robotic surgery system 1 including the robot marker RM installed on the robot, the bone marker BM installed on the bone, and the tracking apparatus 200 for tracking the positions and postures of these markers RM and BM, a transformation matrix for transforming coordinates between the implant coordinate system and the robot coordinate system may be derived using the following Equation 1.

[Equation 1]

$$T^{Robot}_{Implant} = T^{Robot}_{Robot\ marker} \times inv(T^{OTS}_{Robot\ marker}) \times T^{OTS}_{Bone\ marker} \times T^{Bone\ marker}_{Bone} \times T^{Bone}_{Implant}$$

Here,  $T^{Robot}_{Implant}$  is a transformation matrix between the coordinate system of the robot and the coordinate system of the implant,  $T^{Robot}_{Robot\ marker}$  is a transformation matrix between the coordinate system of the robot and the coordinate system of the robot marker RM,  $T^{OTS}_{Robot\ marker}$  is a transformation matrix between the coordinate system of the tracking apparatus 200 and the coordinate system of the robot marker RM,  $T^{OTS}_{Bone\ marker}$  marker is a transformation matrix between the coordinate system of the tracking apparatus 200 and the coordinate system of the bone marker BM,  $T^{Bone\ marker}_{Bone}$  is a transformation matrix between the coordinate system of the bone marker BM and the coordinate system of the bone B, and  $T^{Bone}_{Implant}$  is a transformation matrix between the coordinate system of the bone B and the coordinate system of the implant.

Because it is well known how to derive a transformation matrix for coordinate transformation between heterogeneous coordinate systems during robotic surgery, detailed descriptions thereof will be omitted for simplicity.

The processor 340 may check whether the planned cutting path of the surgical robot 100 belongs to a surgical region set on the surgical robot coordinate system based on the coordinate system transformation matrix calculated as above. The surgical region refers to a region where the surgical robot 100 is accessible and there is no concern about singularity occurring during surgery, and may be preset having various three-dimensional shapes such as a sphere, a rectangular parallelepiped, and a cube, considering the position and posture of the surgical robot 100 and the kinematic range of the surgical robot 100.

When the cutting path defined based on the implant coordinate system does not belong to the surgical region, the processor 340 may provide a notification through the display unit 320 and guide an adjustment direction, etc. for the position and posture of the surgical robot 100 and the position of a patient, etc., to be adjusted so that the cutting path can belong to the surgical region.

As described above, with the cutting path planning apparatus 300 according to the disclosure and the method thereof, a patient-tailored cutting path is automatically created considering the diversity of bone for each patient to thereby improve the surgical effects, and the cutting path is created to minimize the residual bone region remaining after the cutting by the surgical robot to thereby simplify the surgical process and effectively prevent a surgical delay.

Although all elements for an embodiment of the disclosure are combined as one or operating as combined, the disclosure is not limited to this embodiment. In other words, one or more elements among all the elements may be selectively combined and operated as long as they fall into the scope of the disclosure. Further, all the elements may be respectively implemented by independent pieces of hardware, or some or all of the elements may be selectively combined and thus implemented as a computer program having a program module that performs the functions of some or all combinations from one or multiple pieces of hardware. Codes and code segments constituting that computer program may be easily deduced by those skilled in the art. Such a computer program is stored in a computer readable medium and read and executed by a computer, thereby implementing the embodiments of the disclosure. The storage medium of the computer program may include a magnetic recording medium, an optical recording medium, etc.

Further, the terms "comprise," "configure" and/or "have" specify the presence of stated components, unless there is a clearly different meaning in the disclosure, but do not preclude the presence thereof and should be construed to further include other components. Unless otherwise defined, all terms including technical or scientific terms used herein have the same meaning as commonly understood by those skilled in the art to which the disclosure pertains. General terms that are defined in a dictionary shall be construed to have meanings that are consistent in the context of the relevant art, and will not be interpreted as having an idealistic or excessively formalistic meaning unless clearly defined in the disclosure.

The foregoing description is merely an example of technical idea according to the disclosure, and various modifications and changes may be made by a person having ordinary knowledge in the art to which the disclosure pertains without departing from the essential characteristics of the disclosure. Accordingly, the embodiments of the disclosure are not intended to limit but rather to describe the technical idea of the disclosure, and the scope of the technical idea of the disclosure is not limited by these embodiments. The scope of the disclosure should be interpreted based on the appended claims, and all technical ideas within the equivalent scope should be interpreted as falling into the scope of the disclosure.

**Claims**

1.  A method of planning a cutting path of a surgical robot, which are performed by a surgical robot cutting path planning apparatus, the method comprising:

    determining a position and posture of an implant to be installed in a surgical target bone based on a 3D model of a surgical target bone;
    determining a cutting surface of the surgical target bone required to be cut for installation of the implant;
    determining a cutting start position where the surgical robot starts cutting for the cutting surface, and an entrance direction of the surgical robot in the cutting start position;
    setting a maximum region in which the cutting path by the surgical robot is definable based on the cutting start position and the entrance direction;
    generating a modified 3D model in which a virtual cut state of the cutting surface is reflected; and
    creating the cutting path of the surgical robot starting from the cutting start position within a range of the maximum region based on the modified 3D model.

2.  The method of claim 1, further comprising setting a safety region to protect tissue around the surgical target bone, wherein the cutting path is created not to perform cutting in the safety region.

3.  The method of claim 2, wherein

    one or more cutting start positions of the surgical robot are determined for the cutting surface based on skin incision information preset for the cutting of the cutting surface and a location of the safety region, and
    the maximum region and the cutting path are individually determined corresponding to each cutting start position.

4.  The method of claim 1, wherein the cutting path is individually created for each of the cutting surfaces based on the number of cutting surfaces required to be cut for the installation of the implant.

5.  The method of claim 1, further comprising setting a free cutting region that the surgical robot can perform cutting freely based on a location of tissue around the surgical target bone and preset skin incision information.

6.  The method of claim 1, wherein the creating the cutting path of the surgical robot comprises:

    identifying an outer boundary of the cutting surface in the modified 3D model;
    setting a cutting margin region excluded from the cutting by applying a predetermined offset from the outer boundary;
    determining a cutting path creation region to create the cutting path based on the maximum region and the cutting margin region; and
    creating the cutting path with the cutting path creation region served as a boundary.

7.  The method of claim 6, further comprising determining whether the surgical robot can perform cutting for all regions within the cutting path creation region in a predetermined range of postures,
    wherein the creating the cutting path comprises, upon the cutting not performed for all the regions within the cutting path creation region in the predetermined range of postures, creating the cutting path so that the surgical robot can cut some regions within the cutting path creation region, then return to the cutting start position, and complete the cutting by changing the posture and entering the position again.

**8.** The method of claim 6, wherein

one or more cutting start positions of the surgical robot are determined for the cutting surface based on skin incision information preset for the cutting of the cutting surface and a location of the safety region, and
a plurality of cutting path creation regions are determined corresponding to the plurality of cutting start positions, respectively.

**9.** The method of claim 1, wherein the creating the cutting path of the surgical robot comprises determining a return position for the surgical robot to return to the cutting start position after the cutting.

**10.** The method of claim 1, wherein the cutting path is determined considering types of cutting tool of the surgical robot and cutting characteristics of the cutting tool.

**11.** The method of claim 1, wherein the determining the position and posture of the implant comprises determining the position and posture of the implant based on a user input made through a user interface unit.

**12.** A surgical robot cutting path planning apparatus for planning a cutting path of a surgical robot, the apparatus comprising a processor,
the processor being configured to:

determine a position and posture of an implant to be installed in a surgical target bone based on a 3D model of a surgical target bone;
determine a cutting surface of the surgical target bone required to be cut for installation of the implant;
determine a cutting start position where the surgical robot starts cutting for the cutting surface, and an entrance direction of the surgical robot in the cutting start position;
set a maximum region in which the cutting path by the surgical robot is definable based on the cutting start position and the entrance direction;
generate a modified 3D model in which a virtual cut state of the cutting surface is reflected; and
create the cutting path of the surgical robot starting from the cutting start position within a range of the maximum region based on the modified 3D model.

EP 4 591 817 A1

[Fig.1]

[Fig.2]

[Fig.3]

```
                          ┌─────────┐
                          │  Start  │
                          └─────────┘
                               │
                               ▼
        ┌──────────────────────────────────────┐
        │ Plan position and posture of implant  │──── S10
        └──────────────────────────────────────┘
                               │
                               ▼
        ┌──────────────────────────────────────┐
        │      Determine cutting surface of     │
        │         surgical target bone          │──── S20
        └──────────────────────────────────────┘
                               │
                               ▼
        ┌──────────────────────────────────────┐
        │      Set safety region and free       │
        │  cutting region for each cutting      │──── S30
        │             surface                   │
        └──────────────────────────────────────┘
                               │
                               ▼
        ┌──────────────────────────────────────┐
        │       Determine cutting start         │
        │   position, entrance direction        │──── S40
        │   and posture of surgical robot       │
        └──────────────────────────────────────┘
                               │
                               ▼
        ┌──────────────────────────────────────┐
        │       Set maximum region where        │
        │      cutting path can be defined      │──── S50
        └──────────────────────────────────────┘
                               │
                               ▼
        ┌──────────────────────────────────────┐
        │      Generate modified 3D model       │
        │             in which                  │──── S60
        │    virtual cut state is reflected     │
        └──────────────────────────────────────┘
                               │
                               ▼
        ┌──────────────────────────────────────┐
        │  Create cutting path of surgical robot│──── S70
        └──────────────────────────────────────┘
                               │
                               ▼
                          ┌─────────┐
                          │   End   │
                          └─────────┘
```

[Fig.4]

[Fig.5]

[Fig.6]

[Fig.7]

[Fig.8]

```
          ┌─────────┐
          │  Start  │
          └────┬────┘
               │
               ▼
   ┌────────────────────────────┐
   │  Identify outer boundary   │
   │  of cutting surface in     │ ──── S710
   │  modified 3D model         │
   └─────────────┬──────────────┘
                 │
                 ▼
   ┌────────────────────────────┐
   │  Set cutting margin region │
   │  from outer boundary of    │ ──── S720
   │  cutting surface           │
   └─────────────┬──────────────┘
                 │
                 ▼
   ┌────────────────────────────┐
   │  Determine cutting path    │
   │  creation region where     │ ──── S730
   │  cutting path is           │
   │  to be created             │
   └─────────────┬──────────────┘
                 │
                 ▼
   ┌────────────────────────────┐
   │  Create cutting path of    │
   │  surgical robot with       │ ──── S740
   │  cutting path creation     │
   │  region served as boundary │
   └─────────────┬──────────────┘
                 │
                 ▼
          ┌─────────┐
          │   End   │
          └─────────┘
```

[Fig.9]

[Fig.10]

[Fig.11]

[Fig.12]

[Fig.13]

[Fig.14]

## INTERNATIONAL SEARCH REPORT

| International application No. |
| --- |
| **PCT/KR2023/014301** |

**A. CLASSIFICATION OF SUBJECT MATTER**

**A61B 34/10**(2016.01)i; **A61B 34/30**(2016.01)i; **A61B 34/00**(2016.01)i

According to International Patent Classification (IPC) or to both national classification and IPC

**B. FIELDS SEARCHED**

Minimum documentation searched (classification system followed by classification symbols)

A61B 34/10(2016.01); A61B 17/00(2006.01); A61B 17/56(2006.01); A61B 19/00(2006.01); A61B 34/00(2016.01); A61B 34/20(2016.01); A61B 34/30(2016.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수술 로봇(surgical robot), 뼈(bone), 절삭 경로(cutting pathway), 플래닝(planning), 3D 모델(3D model), 임플란트(implant), 절삭면(cutting surface), 절삭 시작 위치(cutting starting position), 진입 방향(introducing orientation), 최대 영역(maximum area), 안전 영역(safety area)

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2021-0097909 A (CUREXO INC.) 10 August 2021 (2021-08-10)<br>See claims 1-11; paragraph [0044]; and figures 1-2. | 1-12 |
| A | KR 10-2020-0066385 A (MAKO SURGICAL CORP.) 09 June 2020 (2020-06-09)<br>See claims 1-5 and 7; and figures 1-9. | 1-12 |
| A | KR 10-2019-0031281 A (MAKO SURGICAL CORP.) 25 March 2019 (2019-03-25)<br>See entire document. | 1-12 |
| A | KR 10-2021-0029322 A (CUREXO INC.) 16 March 2021 (2021-03-16)<br>See entire document. | 1-12 |
| A | KR 10-2022-0072407 A (KIM, Young Tak) 02 June 2022 (2022-06-02)<br>See entire document. | 1-12 |

✓ Further documents are listed in the continuation of Box C.    ✓ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **22 January 2024** | **22 January 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2023/014301**

| **C.** | **DOCUMENTS CONSIDERED TO BE RELEVANT** | |
|---|---|---|
| Category* | Citation of document, with indication, where appropriate, of the relevant passages | Relevant to claim No. |
| A | KR 10-2015-0125069 A (CUREXO INC.) 09 November 2015 (2015-11-09)<br>See entire document. | 1-12 |

Form PCT/ISA/210 (second sheet) (July 2022)

## INTERNATIONAL SEARCH REPORT
### Information on patent family members

International application No.

**PCT/KR2023/014301**

| Patent document cited in search report | | | Publication date (day/month/year) | Patent family member(s) | | | Publication date (day/month/year) |
|---|---|---|---|---|---|---|---|
| KR | 10-2021-0097909 | A | 10 August 2021 | CN | 115038400 | A | 09 September 2022 |
| | | | | EP | 4098218 | A1 | 07 December 2022 |
| | | | | JP | 2023-512006 | A | 23 March 2023 |
| | | | | US | 11701189 | B2 | 18 July 2023 |
| | | | | US | 2023-0079638 | A1 | 16 March 2023 |
| | | | | WO | 2021-153973 | A1 | 05 August 2021 |
| KR | 10-2020-0066385 | A | 09 June 2020 | AU | 2014-374084 | A1 | 21 July 2016 |
| | | | | AU | 2014-374084 | B2 | 12 September 2019 |
| | | | | AU | 2019-275574 | A1 | 02 January 2020 |
| | | | | AU | 2019-275574 | B2 | 06 May 2021 |
| | | | | AU | 2021-203849 | A1 | 08 July 2021 |
| | | | | AU | 2021-203849 | B2 | 27 April 2023 |
| | | | | CA | 2935345 | A1 | 09 July 2015 |
| | | | | CN | 105939692 | A | 14 September 2016 |
| | | | | CN | 105939692 | B | 15 January 2019 |
| | | | | CN | 109938800 | A | 28 June 2019 |
| | | | | CN | 109938800 | B | 16 July 2021 |
| | | | | EP | 3089710 | A1 | 09 November 2016 |
| | | | | EP | 3089710 | B1 | 29 August 2018 |
| | | | | JP | 2017-500964 | A | 12 January 2017 |
| | | | | JP | 2019-155192 | A | 19 September 2019 |
| | | | | JP | 6552505 | B2 | 31 July 2019 |
| | | | | JP | 6877491 | B2 | 26 May 2021 |
| | | | | KR | 10-2016-0104676 | A | 05 September 2016 |
| | | | | KR | 10-2120259 | B1 | 08 June 2020 |
| | | | | KR | 10-2304675 | B1 | 23 September 2021 |
| | | | | US | 10966731 | B2 | 06 April 2021 |
| | | | | US | 2015-0182237 | A1 | 02 July 2015 |
| | | | | US | 2017-0333051 | A1 | 23 November 2017 |
| | | | | US | 2021-0186522 | A1 | 24 June 2021 |
| | | | | US | 9724109 | B2 | 08 August 2017 |
| | | | | WO | 2015-103010 | A1 | 09 July 2015 |
| KR | 10-2019-0031281 | A | 25 March 2019 | AU | 2017-295037 | A1 | 18 January 2018 |
| | | | | AU | 2017-295037 | B2 | 22 July 2021 |
| | | | | AU | 2017-295728 | A1 | 07 February 2019 |
| | | | | AU | 2017-295728 | B2 | 25 March 2021 |
| | | | | AU | 2021-202000 | A1 | 29 April 2021 |
| | | | | AU | 2021-202000 | B2 | 15 September 2022 |
| | | | | AU | 2022-241606 | A1 | 27 October 2022 |
| | | | | CA | 3027143 | A1 | 18 January 2018 |
| | | | | CA | 3030831 | A1 | 18 January 2018 |
| | | | | CN | 109415427 | A | 01 March 2019 |
| | | | | CN | 109688963 | A | 26 April 2019 |
| | | | | CN | 109688963 | B | 06 August 2021 |
| | | | | CN | 113303907 | A | 27 August 2021 |
| | | | | EP | 3484398 | A1 | 22 May 2019 |
| | | | | EP | 3484911 | A1 | 22 May 2019 |
| | | | | EP | 3484911 | B1 | 04 November 2020 |
| | | | | JP | 2019-523049 | A | 22 August 2019 |
| | | | | JP | 2019-524726 | A | 05 September 2019 |

Form PCT/ISA/210 (patent family annex) (July 2022)

**EP 4 591 817 A1**

## INTERNATIONAL SEARCH REPORT
### Information on patent family members

| International application No. |
| --- |
| **PCT/KR2023/014301** |

| Patent document cited in search report | | | Publication date (day/month/year) | Patent family member(s) | | | Publication date (day/month/year) |
| --- | --- | --- | --- | --- | --- | --- | --- |
| | | | | JP | 2022-160618 | A | 19 October 2022 |
| | | | | JP | 2023-015257 | A | 31 January 2023 |
| | | | | JP | 7123031 | B2 | 22 August 2022 |
| | | | | JP | 7177035 | B2 | 22 November 2022 |
| | | | | KR | 10-2022-0157512 | A | 29 November 2022 |
| | | | | KR | 10-2022-0158870 | A | 01 December 2022 |
| | | | | KR | 10-2470282 | B1 | 24 November 2022 |
| | | | | US | 10716630 | B2 | 21 July 2020 |
| | | | | US | 11484368 | B2 | 01 November 2022 |
| | | | | US | 2018-0014891 | A1 | 18 January 2018 |
| | | | | US | 2019-0300567 | A1 | 03 October 2019 |
| | | | | US | 2020-0337784 | A1 | 29 October 2020 |
| | | | | US | 2021-0113278 | A1 | 22 April 2021 |
| | | | | US | 2021-0155655 | A1 | 27 May 2021 |
| | | | | US | 2021-0353367 | A1 | 18 November 2021 |
| | | | | WO | 2018-011381 | A1 | 18 January 2018 |
| | | | | WO | 2018-013848 | A1 | 18 January 2018 |
| KR | 10-2021-0029322 | A | 16 March 2021 | CN | 114364333 | A | 15 April 2022 |
| | | | | EP | 4026509 | A2 | 13 July 2022 |
| | | | | JP | 2022-545743 | A | 28 October 2022 |
| | | | | JP | 7341567 | B2 | 11 September 2023 |
| | | | | KR | 10-2274167 | B1 | 12 July 2021 |
| | | | | US | 11666392 | B2 | 06 June 2023 |
| | | | | US | 2022-0265364 | A1 | 25 August 2022 |
| | | | | WO | 2021-045546 | A2 | 11 March 2021 |
| | | | | WO | 2021-045546 | A3 | 29 April 2021 |
| KR | 10-2022-0072407 | A | 02 June 2022 | None | | | |
| KR | 10-2015-0125069 | A | 09 November 2015 | KR | 10-1570857 | B1 | 24 November 2015 |
| | | | | US | 2015-0305828 | A1 | 29 October 2015 |

Form PCT/ISA/210 (patent family annex) (July 2022)

29